# EUROPEAN PATENT APPLICATION

(11) **EP 1 752 153 A1**
(43) Date of publication of application: **14.02.2007**
(21) Application number: 05737065.2
(22) Date of filing: 06.05.2005
(51) Int. Cl.: A61K 33/00, A61K 33/06, A61K 33/14, A61P 25/02, A61K 35/02

(54) **COMPOSITION HAVING EFFECT OF MODULATING AUTONOMIC NERVES AND METHOD OF USING THE SAME**

(30) Priority: 07.05.2004 JP 2004139245
(71) Applicant: SUNTORY LIMITED, Osaka-shi, Osaka 530-8203 (JP)
(72) Inventor: MORITANI, Toshio, Kyoto-shi, Kyoto 6128034 (JP); SAITO, Kayo, Kyoto-shi, Kyoto 6060855 (JP)
(74) Representative: Vossius & Partner
(86) International application number: PCT/JP2005/008356
(87) International publication number: WO 2005/107775

(57) **Abstract**

The present invention provides a composition capable of regulating the activity of autonomic nerves, by acting on these nerves, so as to maintain the homeostasis or alleviate disturbed homeostasis in an animal, wherein the above composition can be obtained from a naturally occurring substance in the nature by a simple process.

The seawater mineral-containing composition of the present invention is **characterized in that** it contains seawater minerals obtained by treating seawater and is capable of regulating autonomic nerves. The seawater mineral-containing composition of the present invention preferably has a magnesium concentration of 70 mg/L or higher and a sodium concentration of 3 mg/L or lower.

## Description

### TECHNICAL FIELD

The present invention relates to a seawater mineral-containing composition, and foods and/or drinks containing the same.

### BACKGROUND ART

In order to maintain homeostasis, a living body has autonomic nervous system, endocrine system, and immune system. Of these systems, the autonomic nervous system is relatively independent from the control by the cerebrum, and it autonomously functions irrelevantly to the intention. Thus, this system is named "autonomic nervous system." It mainly regulates the functions of internal organs. The autonomic nervous system is divided into the two systems, sympathetic nervous system and parasympathetic nervous system. Thus, the autonomic nervous system is controlled by the balance between the above two systems. That is to say, when a body is active, the activity of sympathetic nerves is dominant and the body as a whole is in a state of tension. In contrast, when the activity of parasympathetic nerves is dominant, the body is released from tension and becomes a relaxed state.

When the activity of sympathetic nerves systemically increases, hormone secretion from the adrenal medulla is promoted, resulting in an increase in heart rate or blood pressure, bronchiolar dilation, suppression of gastrointestinal movement and secretion, increase in the glucose metabolism, mydriasis, piloerection, contraction of the skin or blood vessels in internal organs, and dilation of blood vessels in the skeletal muscle (Iwanami Koza, "Gendai Igaku no Kiso (Basics of Modern Medicine)" Vol. 4, edited by Toshio Hagiwara and Seiichiro Tarui, Seitai no Cyosetsu System (Regulation Systems in the Living Body), 1999). Accordingly, if the activity of sympathetic nerves is enhanced, it is likely to give rise to health disturbances such as hypertension, hyperglycemia, reduced blood flow in the skin, or decreased immunological function. On the other hand, if the activity of parasympathetic nerves is enhanced, it is likely to give rise to chronic diarrhea. In addition, it has been reported that a decrease in the activity of parasympathetic nerves and a corresponding relative increase in the activity of sympathetic nerves are significant risk factors which are responsible for coronary artery disease or sudden death (Akselrod et al., Science 213: 220-22 (1981), Billman GE et al., Circulation 80: 874-80 (1989)). Unbalance between the autonomic nerves occurs also due to stress. Thus, people often suffer from poor physical conditions of unknown causes.

Under such circumstances, pharmaceuticals or health food products for controlling the activity of autonomic nerves have been desired, and a number of pharmaceuticals have already been placed on the market.

Water obtained by treating seawater is rich in mineral components. Products containing mineral components derived from seawater have been developed and become a focus of attention. In addition, various applications of such products have been disclosed (Japanese Patent Laid-Open Nos. 2000-295974, 2001-136942, 2001-211864, 2001-87762, etc.). However, although various actions of seawater have been reported, the effects on health which will be obtained by ingesting a beverage from seawater, and in particular, the effect or the regulatory action of such a beverage on the activity of the autonomic nervous system have never been studied or reported until today.
Patent document 1: Japanese Patent Laid-Open No. 2000-295974
Patent document 2: Japanese Patent Laid-Open No. 2001-136942
Patent document 3: Japanese Patent Laid-Open No. 2001-211864
Patent document 4: Japanese Patent Laid-Open No. 2001-87762 Non-patent document 1: Iwanami Koza, "Gendai Igaku no Kiso (Basics of Modern Medicine)" Vol. 4, edited by Toshio Hagiwara and Seiichiro Tarui, Seitai no Cyosetsu System (Regulation Systems in the Living Body
Non-patent document 2: Akselrod et al., Science 213: 220-22 (1981)
Non-patent document 3: Billman GE et al., Circulation 80: 874-80 (1989)

### DISCLOSURE OF INVENTION

### PROBLEMS TO BE SOLVED BY THE INVENTION

In the case of pharmaceuticals, it has previously been difficult to achieve both the control on autonomic nerves and low side effects. In addition, multiple processes have been required to synthesize pharmaceuticals. Moreover, for easy ingestion, such pharmaceuticals have to be processed into a form such as a tablet. As a result, the high cost of production has been a problem of the conventional pharmaceuticals. Thus, it is strongly desired to discover an active agent from a naturally occurring substance in the nature by a simple process. It is an object of the present invention to provide a composition capable of regulating the activities of the autonomic nervous system, by acting on autonomic nerves, in order to maintain the homeostasis or normalize disturbed homeostasis of an animal.

As a result of extensive studies directed towards achieving the aforementioned object, the present inventors have found that a seawater mineral-containing composition is effective for maintaining the homeostasis of an animal. That is to say, the composition of the present invention is a seawater mineral-containing composition, which is characterized in that it comprises seawater minerals obtained by treating seawater and that it is effective for regulating the autonomic nervous system. The seawater mineral-containing composition of the present invention preferably has a magnesium concentration of 70 mg/L or higher, and a sodium concentration of 6 mg/L or lower, and particularly preferably 3 mg/L or lower.

### EFFECTS OF THE INVENTION

Ingestion of the seawater mineral-containing composition enables suppression of enhanced activity of sympathetic nerves and enhancement of a decreased activity of parasympathetic nerves. The action of regulating autonomic nerves is extremely useful not only for prevention and/or alleviation of a state of unbalance in the autonomic nervous system, but also extremely useful against various diseases which often develop in a state where the activity of sympathetic nerves is dominant.

### BRIEF DESCRIPTION OF THE DRAWINGS

[Fig.1] Figure 1 shows the effect of ingestion of the seawater mineral-containing composition in suppressing the index of sympathetic nerve activity.
[Fig.2] Figure 2 shows the effect of ingestion of the seawater mineral-containing composition in enhancing the activity of parasympathetic nerves.
[Fig.3] Figure 3 shows the effect of ingestion of the seawater mineral-containing composition in suppressing the extension of RT.

### BEST MODE FOR CARRYING OUT THE INVENTION

The method of obtaining the seawater mineral-containing composition of the present invention is not particularly limited. Thus, the composition can be obtained by the method described in Japanese Patent Laid-Open No. 2004-065196, for example.

Types of seawater which may be employed as a starting material for producing the seawater mineral-containing composition include surface sea water, intermediate sea water, and deep-sea water. Of these, deep-sea water, and particularly, sea water from a depth of 200 m or more, is excellent in cleanliness, since it is not affected by environmental pollution. Moreover, deep-sea water is rich in mineral components as they are less consumed by marine organisms. Thus, deep-sea water is preferably used in the present invention.

With regard to the contents of mineral components in the seawater mineral-containing composition, it is preferable that the ratio of the mineral components having beneficial effects on health, such as magnesium or calcium, be high, and that the concentration of sodium be low. A magnesium concentration of 70 mg/L or higher is preferred. A sodium concentration of 6 mg/L or lower, and in particular 3 mg/L or lower, is preferred.

In order to obtain such a composition, an electrodialysis method comprising subjecting seawater to electrodialysis using a monovalent cation-selective dialysis membrane is preferably employed. As a monovalent cation-selective dialysis membrane, AC120 (manufactured by Asahi Kasei Corp.) or the like can be used. A common electrodialysis device may be used and the electrodialysis is carried out such that the electric conductivity of the water after completion of the electrodialysis has been lowered to such a level as less than 10 mS/cm. In this way, the sodium concentration can be reduced, and the magnesium concentration can be increased, thereby to produce a seawater mineral-containing composition having a consistent mineral composition. Taking the cost of water and electric power used into consideration, the electric conductivity after completion of the electrodialysis will be preferably 8 mS/cm or less, and particularly preferably 6 mS/cm. When the electric conductivity after completion of the electrodialysis is as low as 6 mS/cm, for example, it is possible to produce a seawater mineral-containing composition that has a sodium concentration of 4 mg/L or lower, a magnesium concentration of 20 mg/L or higher, and the weight ratio between magnesium and calcium is 4 or greater, by adjusting the hardness of the aqueous solution from the electrodialysis to 100 as determined by the EDTA method. The weight ratio between magnesium and calcium of 4 or greater is advantageous in that magnesium can be conveniently administered to people as they are not takings sufficient amount of magnesium these days.

When it is desired to produce a seawater mineral-containing composition having a magnesium concentration of 70 mg/L or higher and a sodium concentration of 3 mg/L or lower, in order to use it to effectively exert its autonomic nerve-regulating action, the following multistage electrodialysis method can be employed, for example. That is, as a first electrodialysis, seawater is electrodialyzed by using a monovalent cation-selective dialysis membrane, until the electric conductivity of the seawater becomes 12 mS/cm, as in the case of the common salt-manufacturing method, or lower. At the end of the first electrodialysis, the magnesium concentration will be approximately 1300 mg/L, and the sodium concentration will be approximately 640 mg/L. The mineral water obtained from the first electrodialysis is subjected to a second electrodialysis using again the monovalent cation-selective dialysis membrane, until the electric conductivity becomes 6 mS/cm or lower. The mineral water obtained from the second electrodialysis has a magnesium concentration of approximately 740 mg/L and a sodium concentration of approximately 2 mg/L. Thus, this mineral water is appropriately diluted with seawater containing a decreased amount of sodium, such as RO (reverse osmosis) treated seawater, to prepare mineral water with a hardness of approximately 330 (EDTA method), thereby obtaining the seawater mineral-containing composition of the present invention having preferable magnesium and sodium concentrations.

Moreover, it is possible to more extensively remove monovalent ions such as sodium or potassium ions from the seawater mineral-containing composition of the present invention having already preferable magnesium and sodium concentrations. This may be achieved by maintaining the sodium concentration to be constantly low in the chamber on the concentration side of an electrodialysis device, thereby preventing back diffusion of sodium. In this case, it is preferable to perform electrodialysis while maintaining the sodium concentration in the chamber on the concentration side at 20 mg/L or lower, and preferably at 2 mg/L or lower.

The seawater mineral-containing composition of the present invention has an excellent flavor and also has an extremely low sodium concentration. Accordingly, with regard to application to foods and drinks, the present composition can be applied to various products in general. By adding the present composition to foods and drinks, the amounts of mineral components such as magnesium or calcium in the foods and drinks can be controlled. The amount of the seawater mineral-containing composition of the present invention to be added can be determined depending on the forms of foods and drinks to which the composition is applied. For example, a product can be designed taking the dose of magnesium as an index. In that case, the dose of magnesium for a single ingestion can be chosen from the range between 1 mg and 700 mg. The dose of magnesium per day from the seawater mineral-containing composition of the present invention is preferably 100 mg or more. Since the seawater mineral-containing composition of the present invention contains magnesium at a concentration of 70 mg/L. or more, magnesium can be ingested effectively from the composition.

Moreover, since the seawater mineral components in the composition of the present invention are very stable, the seawater mineral-containing composition itself, or foods and drinks containing the seawater mineral-containing composition, may be subjected to treatments such as heating, cooling, or freezing. With regard to the forms of the seawater mineral-containing composition, it may directly be used as mineral water, or it may be converted into forms such as a dry product, a concentrated product, or a diluted product. For example, the seawater mineral-containing composition may be formulated as a supplement product such as in the form of beverage, capsule, tablet, powder or jelly. Specific examples of such products include fruit juice, soft drink, sour milk beverage, carbonated drink, coffee beverage, tea beverage, vegetable juice, liqueur, distilled spirit-mixed drink, distilled spirit, wine, beer, tablet, candy, oleaster, cookie, and jelly. Further, additives such as vitamins, polyphenols, amino acids, peptides, proteins, sugars, or organic acids may be added to these products before use.

The activity of autonomic nerves of a person can be regulated by ingesting the seawater mineral-containing composition of the present invention. For example, the seawater mineral-containing composition prepared from the treated seawater as described above is ingested, and the activity of his autonomic nerves can then be evaluated by analyzing the power spectrum of his heart rate variability. It will be found that the activity of sympathetic nerves is suppressed and that the activity of parasympathetic nerves is enhanced by the ingestion of the composition. In addition, it will also be found by analyzing the electrocardiogram that the time intervals required for cardiac depolarization and repolarization (Recovery Time: RT) is reduced by the ingestion of the composition. That is, the seawater mineral-containing composition of the present invention is effective for regulating the activity of autonomic nerves.

Methods for evaluating the effect of the seawater mineral-containing composition of the present invention on the activity of autonomic nerves include: biophysical measurement methods such as electrocardiography, measurement of blood pressure, galvanic skin response, or pupil diameter; and biochemical measurement methods such as measurement of catecholamine level in blood, and so on.

Of these methods, a preferred method for evaluating the effect on the activity of autonomic nerves is to observe heart rate variability, since said variability is caused by the action of autonomic nerves on the heart. For example, it is possible to evaluate the effect of the seawater mineral-containing composition in the alleviation of disturbed activity of autonomic nerves by analyzing the power spectrum of heart rate variability and the recovery time (RT), used as indexes. More specifically, R-R intervals in electrocardiogram (heart beat rhythms, that are, intervals between heat beats (R-R *intervals)* vary constantly, and such variability in R-R intervals is called heart rate variability) are converted into power spectrum by fast Fourier transformation. Thereafter, the integral value of the spectrum is obtained from a low frequency component power (LOW) with frequencies between 0.03 and 0.15 Hz, a high frequency component power (HIGH) with frequencies between 0.15 and 0.4 Hz, and the sum total thereof (TOTAL). The activity of autonomic nerves can be then evaluated, using LOW/HIGH as an index of the activity of sympathetic nerves, and using HIGH/TOTAL as an index of the activity of parasympathetic nerves. That is to say, when the activity of cardiac sympathetic nerve is suppressed, the value of LOW/HIGH decreases, and when the activity of cardiac parasympathetic nerve is enhanced, the value of HIGH/TOTAL increases.

RT obtained by an analysis of electrocardiogram reflects the time required for repolarization of the ventricular muscle, that is, the retention time of the intracellular action potential. Thus, RT is an important index that reflects the refractory period of the ventricular muscle. A repolarization phase in the cardiac muscle sharply responds to various pathologic factors in daily life, such as heart rate, autonomic nerve activity, serum electrolyte, ischemia, or administration of drugs.

### Examples

The present invention is described in detail with reference to the following examples. However, these examples are not intended to limit the present invention.

### [Example 1]

### Preparation example 1 of seawater mineral-containing composition

Seawater obtained from the depth of 330 m (off the coast of Misaki, Miura Peninsula, Sizuoka Prefecture, Japan) was subjected to electrodialysis using an electrodialysis device (SV1/2 type), manufactured by Asahi Kasei Corp., until the electric conductivity of the seawater became 12 mS/cm when the electrodialysis was terminated, so as to obtain first mineral water.

The first mineral water was then subjected to electrodialysis using an electrodialysis device (S3 type), Asahi Kasei Corp., until the electric conductivity became 6 mS/cm, so as to obtain second mineral water. As the electrodialysis membrane, Asahi Kasei type AC120 was used for the production of both the first and the second mineral water. The electrodialysis for producing the second mineral water was carried out under conditions consisting of a preset temperature at the starting time of 15°C, an electric conductivity in the chamber on the concentration side of 1.5 mS/cm, a circulation flow rate of 1.4 L/min., and a constant voltage of 12.5 V.

The thus obtained second mineral water was diluted with desalted water (with a sodium concentration = 1.8 mg/L), which had been obtained by treating seawater from the depth of 330 m by two steps using reverse osmosis membranes SW30HR-380 (high pressure) and SWLE-440 (low pressure) manufactured by The Dow Chemical Company, thereby to produce a seawater mineral-containing composition with a hardness of 330 (EDTA method). The composition is shown in [Table 1]

**Table 1: Seawater mineral-containing composition (per liter) (hardness: 330 (EDTA method))**

| | |
|---|---|
| Energy, protein | 0 |
| Lipid, carbohydrate | 0 |
| Sodium | 2.6 mg |
| Magnesium | 75.2 mg |
| Calcium | 11.0 mg |
| Potassium | 58.6 µg |
| Iodine | 0.84 µg |
| Phosphorus | 5.26 µg |
| Copper | 0.18 µg |
| Selenium | 1.40 µg |
| Zinc | 0.54 µg |
| Molybdenum | 0.08 µg |

### [Example 2]

### Regulating effect on autonomic nerve activity

This test was carried out on 21 adult males. The seawater mineral-containing composition (hardness: 330 mg/L) obtained in Example 1 was given to the subjects in an amount of 1.5 L per day for 5 weeks. At the time points when the ingestion was initiated and terminated, electrocardiograms at rest of the subjects were measured. Thereafter, the effect of the above seawater mineral-containing composition on the activity of autonomic nerves was examined by the power spectrum analysis of heart rate variability in accordance with the method of Ue, H et al. (Ann. Noninvasive Electrocardiol., 5: 336-345 (2000)), i.e, the time required for depolarization and repolarization of the ventricular muscle (RT) was examined by linear differential analysis of the electrocardiogram.

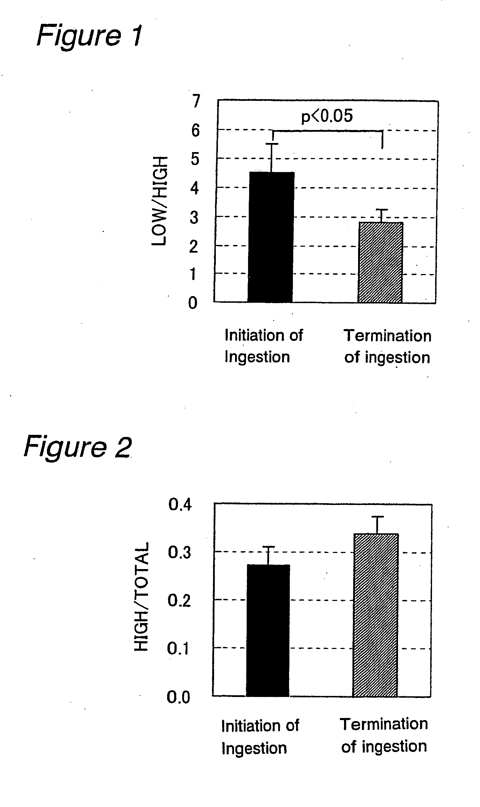

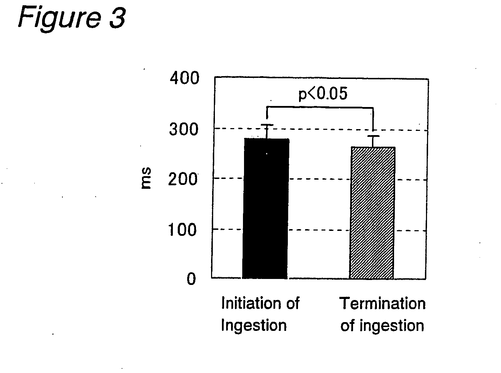

Namely, electrocardiogram sampling was carried out at 1024 Hz by 14-bit A/D conversion. The heart rate variability obtained from the electrocardiogram was processed by fast Fourier transformation, and frequency analysis was conducted to obtain a power spectrum of the heart rate variability. In order to separate and quantify the heart rate variability spectrum, integral values of the spectra were obtained from the low frequency component power (LOW) with frequencies between 0.03 and 0.15 Hz, the high frequency component power (HIGH) with frequencies between 0.15 and 0.4 Hz, and the sum total thereof (TOTAL). LOW/HIGH was used as an index of the activity of sympathetic nerves, and HIGH/TOTAL was used as an index of the activity of parasympathetic nerves. The obtained results are shown in Figures 1 and 2. Also, the time required for cardiac depolarization and repolarization was studied from RT. The obtained results are shown in Figure 3.

## Claims

1. A seawater mineral-containing composition capable of regulating autonomic nerves comprising seawater minerals obtained by treating seawater.

2. The seawater mineral-containing composition according to claim 1, wherein magnesium is contained in an amount of 70 mg/L or higher, and sodium is contained in an amount of 6 mg/L or lower.

3. The seawater mineral-containing composition according to claim 1, wherein the seawater derives from deep-sea, and wherein the composition is obtained by treating the seawater with a dialysis membrane which is selective for monovalent cations, thereby to provide an electric conductivity of the treated seawater of less than 10 mS/cm.

4. The seawater mineral-containing composition according to any one of claims 1 to 3, which is capable of regulating autonomic nerves to prevent, palliate or alleviate a disturbed state of autonomic nerves and/or symptoms caused thereby.

5. The composition according to claim 4, which is used to suppress an enhanced state of the activity of sympathetic nerves by ingesting seawater minerals.

6. The composition according to claim 4, which is used to enhance the activity of parasympathetic nerves by ingesting seawater minerals.

7. The composition according to claim 4, which is used to reduce the time intervals between cardiac depolarization and repolarization by ingesting seawater minerals.

8. The composition according to any one of claims 4 to 7, which is suitable for ingesting magnesium at an amount of 100 mg or higher per day.

9. The composition according to claim 1, which is in the form of food or drink or a component thereof.
